# EUROPÄISCHE PATENTSCHRIFT

(11) **EP 3 806 820 B1**
(45) Veröffentlichungstag und Bekanntmachung des Hinweises auf die Patenterteilung: **03.06.2026**
(21) Anmeldenummer: 19731948.6
(22) Anmeldetag: 14.06.2019
(51) Int. Cl.: A61K 8/99, A61P 17/10, A61Q 19/00, A61K 35/744

(54) **KOSMETISCHE ZUBEREITUNG GEGEN AKNE**
COSMETIC PREPARATION AGAINST ACNE
PRÉPARATION COSMÉTIQUE CONTRE L'ACNÉ

(30) Priorität: 14.06.2018 DE 102018209519
(43) Veröffentlichungstag der Anmeldung: 21.04.2021
(73) Patentinhaber: Beiersdorf AG, 22529 Hamburg (DE); S-Biomedic NV, 2340 Beerse (BE)
(72) Erfinder: RICHTER, Daniel, 22850 Norderstedt (DE); HÜPEDEN, Jennifer, 20251 Hamburg (DE); REUTER, Jörn Hendrik, 24558 Henstedt Ulzburg (DE); FÖLSTER, Heike, 22149 Hamburg (DE); FELTEN, Bernhard, 25421 Pinneberg (DE); DJAMIL, Jane, 20357 Hamburg (DE)
(74) Vertreter: Eisenführ Speiser
(86) Internationale Anmeldenummer: PCT/EP2019/065783
(87) Internationale Veröffentlichungsnummer: WO 2019/238968

(56) Entgegenhaltungen:
- WO-A1-2016/172196
- WO-A1-2018/073651
- WO-A2-2017/136738
- US-A1- 2017 065 647
- CHRISTIAN F. P. SCHOLZ ET AL: "A Novel High-Resolution Single Locus Sequence Typing Scheme for Mixed Populations of Propionibacterium acnes In Vivo", PLOS ONE, vol. 9, no. 8, 11 August 2014 (2014-08-11), pages e104199, XP055460938, DOI: 10.1371/journal.pone.0104199

## Beschreibung

Die vorliegende Erfindung betrifft ein kosmetisches Produkt aus einem Packmittel mit zwei räumlich getrennten Kammern, wobei die erste Kammer ein Lyophyllisat aus Bakterien der Stämme *C*. *acnes* 6609 (H1), C1, C3, D1, A5, H1, H2, H3, K1, K2, K4, K6, K8, K9, L1 und/oder F4 enthält und die zweite Kammer eine Wasser enthaltende kosmetische Zubereitung in Form einer Öl-in-Wasser Emulsion enthält sowie ein Verfahren zur Behandlung von Akne mit dieser Zubereitung.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht. Einen wesentlichen Anteil an einem schönen und attraktiven Äußeren hat dabei der Zustand und das Aussehen der Haut.

Bei fettig-öliger und unreiner Haut, als welche man den Übergangszustand zwischen der gesunden normalen und der krankhaft veränderten Aknehaut bezeichnet, produziert die Haut erhöhte Mengen an Talg (Seborrhoe). Dieser dient zahlreichen Mikroorganismen, insbesondere *Propionibacterium acnes* (heutzutage als Cutibacterium acnes, *C*. *acnes* bezeichnet, weshalb im Rahmen dieser Offenbarung die neue Abkürzung *C*. *acnes* an Stelle von *P*. *acnes* verwendet wird) und *Pityrosporum*-Arten als idealer Nährboden. Die Mikroorganismen zersetzen den Talg zu Glycerin und Fettsäuren, wodurch die Talgdrüsen zu erhöhter Produktion angeregt und die Follikelwandungen in der Haut angegriffen und zerstört werden. Dies ruft Entzündungen in der Haut (Pickel, Pusteln, Knoten, Zysten) hervor, welche oft nur narbig ausheilen, wodurch das optische Erscheinungsbild des an unreiner Haut leidenden Menschen dauerhaft geschädigt wird (W. Umbach [Hrsg], Kosmetik, Entwicklung, Herstellung und Anwendung kosmetischer Mittel, 2.Aufl. Thieme Verlag, Stuttgart, 1995).

Unter Akne (im engeren Sinne *acne vulgaris*) versteht man verschiedene Erkrankungen der Talgdrüsenfollikel, die durch Sekretions- und Verhornungsstörungen nachfolgende Entzündungen und eventuelle Vernarbung gekennzeichnet sind. Die *acne vulgaris* tritt hauptsächlich in der Pubertät auf und konzentriert sich in der Regel auf talgdrüsenreiche Hautbezirke (Gesicht, Nacken, Brust, Rücken). Durch Talgdrüsenhyperplasie und eine Verhornungsstörung der Follikel kommt es zu deren Verstopfung mit Bildung von Komedonen und den für *acne vulgaris* typischen Effloreszenzen (Pschyrembel, Klinisches Wörterbuch, 258. Aufl., Walter de Gruyter-Verlag, Berlin, 1998).

Herkömmliche Produkte zur Behandlung fettiger und/oder unreiner Haut sowie von AkneHaut haben in der Regel den Nachteil die Haut zu strapazieren, auszutrocknen und wenig pflegend zu wirken.

Bei der Behandlung der Akne werden darüber hinaus stark saure (pH-Werte kleiner pH 4,0) sowie stark oxidierende Wirkstoffe (z.B. Benzoylperoxid) eingesetzt, welche den Säureschutzmantel der Haut strapazieren und die Haut angreifen bzw. verätzen.

US 2017/065647 A1 beschreibt die Behandlung von Akne durch topische Applikation lebender Stämme von *Cutibacterium acnes.* WO 2017/136738 A2 beschreibt drei Stämme von *Cutibacterium acnes*, die gemäß SLST-Schema als K1 zu klassifizieren sind und gesunder Haut zugeordnet werden können.

Es war daher die Aufgabe der vorliegenden Erfindung eine deutlich mildere und besser pflegende kosmetische und/oder dermatologische Zubereitung zur Behandlung fettiger und unreiner Haut sowie zur Behandlung von Akne (und insbesondere *acne vulgaris*) zu entwickeln, als sie vom Stand der Technik her bekannt sind. Ferner war es die Aufgabe der vorliegenden Erfindung, eine kosmetische und/oder dermatologische Zubereitung zur Behandlung von Akne (und insbesondere *acne vulgaris*) zu entwickeln, die bei einem hautfreundlichen pH-Wert (pH größer pH 4,0) wirksam ist, die Haut nicht verätzt und den Säureschutzmantel der Haut allenfalls gering belastet. Es sollten ferner Zubereitungen entwickelt werden, die bei einem hautfreundlichen pH-Wert (pH größer pH 4,0) das Wachstum und die Verbreitung von Akne hervorrufenden Bakterien auf der Haut unterdrücken. Ferner sollte die Konzentration an Akne hervorrufenden Bakterien auf der Haut und in den Talgdrüsen durch diese Zubereitungen signifikant reduziert werden.

Überraschend gelöst werden die Aufgaben durch ein kosmetisches Produkt aus einem Packmittel mit zwei räumlich getrennten Kammern, wobei die erste Kammer ein Lyophyllisat aus Bakterien der Stämme *C*. *acnes* 6609 (H1), C1, C3, D1, A5, H1, H2, H3, K1, K2, K4, K6, K8, K9, L1 und/oder F4 enthält und die zweite Kammer eine Wasser enthaltende kosmetische Zubereitung in Form einer Öl-in-Wasser Emulsion enthält, wobei die die kosmetische Zubereitung in der zweiten Kammer Glycerylstearatcitrat, Cetearylalkohol, Natriumcetearylsulfat, Glycerylstearat, Cetearylsulfosuccinat, Natriumstearoylglutamat, Polyglyceryl-3-methylglucosedistearat, Stearinsäure, Kaliumcetylphosphat, Polyglyceryl-10 Stearat (INCI Polyglyceryl-10 Stearate) und/oder Polyclyceryl-2 Caprat enthält.

Dabei kann das Lyophyllisat aus Bakterien der Stämme *C*. *acnes* 6609 (H1), C1, C3, D1, A5, H1, H2, H3, K1, K2, K4, K6, K8, K9, L1 und/oder F4 nach einem der herkömmlichen Verfahren des Standes der Technik zur Lyophilisation von Bakterien (beispielsweise unter Zusatz von Dextran und/oder Glycerin) hergestellt werden, die den Standardwerken der pharmazeutischen Technologie entnommen werden können.

Zwar kennt der Stand der Technik die WO 2016/172196 und WO 2018/073651, welche auch die erfindungsgemäßen *C*. *acnes* Stämme offenbaren, doch konnte diesen Schriften nicht den Weg zur vorliegenden Erfindung weisen, weil u.a. nicht offenbart ist, mit welchem Medium die C. *acnes* Bakterien in ausreichender Menge aktiviert werden können. Zwar wird in der WO 2016/172196 auf die grundsätzliche Möglichkeit eines Einsatzes von gefriergetrockneten Bakterien hingewiesen, doch bereitete es dem Fachmann große Schwierigkeiten, diese in medizinisch unbedenklichen, jedoch wirksamen Mengen in kurzer Zeit auf der Haut wieder zu aktivieren. Auch ist nicht offenbart, mit welchen Mitteln die Bakterien in auch für Laien einfach handhabbarer Weise auf die betroffenen Hautpartien gezielt und in gleichmäßiger (homogener) Konzentration aufgetragen werden können, denn nicht jedes Kosmetikum ist hierfür ein geeigneter Träger. Phasentrennungen, Agglomeratbildung, Abscheidungen aus der Zubereitung, Viskositätsänderungen sind typische Probleme, die bei der Einarbeitung von Lyophylisaten auftreten. Nicht zuletzt enthalten praktisch alle Kosmetika Inhaltsstoffe, welche diese vor mikrobieller Zersetzung schützen sollen, so dass bei einer einfachen Kombination von kosmetischer Zubereitung und Bakterien davon auszugehen war, dass letztere durch die konservierenden Bestandteile der kosmetischen Zubereitung mehr oder weniger vollständig abgetötet werden.

Die erste Kammer kann neben dem Lyophyllisat aus Bakterien ggf. kosmetische Bestandteile wie Öle und/oder Verdicker enthalten. Auch kann es erfindungsgemäß von Vorteil sein, wie in der WO 2018/073651 vorgeschlagen, Pepton und/oder Hydroxyethylcellulose zuzusetzen.

Erfindungsgemäß bevorzugt ist es, die *C*. *acnes* Stämme C3 und/oder K8 einzusetzen. Dabei ist die Kombination aus beiden Stämmen zu gleichen Anteilen, erfindungsgemäß besonders bevorzugt.

Erfindungsgemäß besonders bevorzugt ist es, diese Stämme (d.h. C3 und/oder K8) mit *C*. *acnes* Stämmen A5 und/oder F4 zu kombinieren.

Es ist erfindungsgemäß von Vorteil, wenn die kosmetische Zubereitung in der zweiten Kammer einen pH-Wert von 4 bis 8 aufweist.

Um dem erfindungsgemäßen Produkt die für die Anwendung richtige Konsistenz zu geben, ist es erfindungsgemäß von Vorteil, wenn die die kosmetische Zubereitung in der zweiten Kammer ein oder mehrere Verdickungsmittel enthält.

Dabei ist es insbesondere vorteilhaft im Sinne der vorliegenden Erfindung, wenn die kosmetische Zubereitung in der zweiten Kammer eine Viskosität von mindestens 500 mPas aufweist. Die Viskositäten werden erfindungsgemäß gemessen mit dem Gerät Rheomat R 123 mit der Spindel Messkörper 1 und der konstanten Drehzahl von 62,5 pro min bei der Temperatur 25°C.

Erfindungsgemäß bevorzugt ist es, wenn die kosmetische Zubereitung in der zweiten Kammer Carageenan und/oder Polyacrylsäure enthält. Dabei umfasst der Begriff Polyacrylsäure erfindungsgemäß auch die Salze (insbesondere das Natriumsalz) dieser Verbindung.

Enthält die Zubereitung Carageenan so ist eine Konzentration von 0,01 bis 1 Gewichts-%, bezogen auf das Gesamtgewicht der kosmetischen Zubereitung in der zweiten Kammer erfindungsgemäß bevorzugt.

Enthält die Zubereitung Polyacrylsäure (Carbomer) so ist eine Konzentration von 0,01 bis 1 Gewichts-%, bezogen auf das Gesamtgewicht der kosmetischen Zubereitung in der zweiten Kammer erfindungsgemäß bevorzugt.

Hingegen ist es erfindungsgemäß von Nachteil, die Zubereitung in der zweiten Kammer mit Xanthangummi zu verdicken, weil es in diesem Falle zur Agglomerat-Bildung und Verklumpung in der Zubereitung kommen kann.

Daher sind vorteilhafte Ausführungsformen der vorliegenden Erfindung dadurch gekennzeichnet, dass die kosmetische Zubereitung in der zweiten Kammer frei ist von Xanthangummi.

Es ist erfindungsgemäß von Vorteil, wenn kosmetische Zubereitung in der zweiten Kammer frei ist von Zubereitung frei ist von Parabenen, Methylisothiazolinon, Chlormethylisothiazolinon, 3-Iod-2-propinylbutylcarbamat, DMDM-Hydantoin, 2-Hydroxy-4-methoxybenzophenon, 4-Methoxyzimtsäure(2-ethylhexyl)ester 4-Methoxyzimtsäureisoamylester, 3-(4-Methylbenzyliden)campher.

Generell ist es erfindungsgemäß bevorzugt, wenn die kosmetische Zubereitung in der zweiten Kammer frei ist von antimikrobiell wirksamen Substanzen. Daher wird die die zweite Kammer des erfindungsgemäßen Produktes erfindungsgemäß vorteilhaft so ausgestaltet, dass die Befüllung unter sterilen Bedingungen erfolgt, die Kammer anschließend luftdicht verschlossen wird und ihr Inhalt mit Hilfe einer Entnahmevorrichtung durch eine einmalige Entnahme (Einmalanwendung) dem Vorratsbehältnis entnommen und nach der Vermischung mit dem Lyophyllisat der ersten Kammer unmittelbar zur Anwendung kommt.

Erfindungsgemäß vorteilhafte Ausführungsformen der vorliegenden Erfindung sind auch dadurch gekennzeichnet, dass die kosmetische Zubereitung in der zweiten Kammer Glycerin, Panthenol, Ubichinon Q10 und/oder Hyaluronsäure enthält.

Enthält die Zubereitung Glycerin so ist eine Konzentration von 1 bis 10 Gewichts-%, bezogen auf das Gesamtgewicht der kosmetischen Zubereitung in der zweiten Kammer erfindungsgemäß bevorzugt.

Enthält die Zubereitung Panthenol so ist eine Konzentration von 0,01 bis 5 Gewichts-%, bezogen auf das Gesamtgewicht der kosmetischen Zubereitung in der zweiten Kammer erfindungsgemäß bevorzugt.

Enthält die Zubereitung Hyaluronsäure so ist eine Konzentration von 0,01 bis 2 Gewichts-%, bezogen auf das Gesamtgewicht der kosmetischen Zubereitung in der zweiten Kammer erfindungsgemäß bevorzugt.

Enthält die Zubereitung Ubichinon Q10 so ist eine Konzentration von 0,01 bis 0,05 Gewichts-%, bezogen auf das Gesamtgewicht der kosmetischen Zubereitung in der zweiten Kammer erfindungsgemäß bevorzugt.

Darüber hinaus kann die kosmetischen Zubereitung in der zweiten Kammer erfindungsgemäß bevorzugt entzündungshemmende Substanzen enthalten, beispielsweise Polidocanol, Bisabolol, aloe vera-Extrakt, Kamillenblütenextrakt, Glycyrrhiza Inflata Root Extract.

Erfindungsgemäß vorteilhaft ist für die kosmetische Zubereitung in der zweiten Kammer ein Wassergehalt bis 100 Gewichts-%, bezogen auf das Gesamtgewicht der der kosmetischen Zubereitung in der zweiten Kammer.

Es ist erfindungsgemäß von Vorteil, wenn die kosmetische Zubereitung in der zweiten Kammer Salicylsäure, Milchsäure und/oder Zitronensäure enthält.

Enthält die Zubereitung Salicylsäure so ist eine Konzentration von 0,01 bis 2 Gewichts-%, bezogen auf das Gesamtgewicht der kosmetischen Zubereitung in der zweiten Kammer erfindungsgemäß bevorzugt.

Enthält die Zubereitung Milchsäure so ist eine Konzentration von 0,01 bis 3 Gewichts-%, bezogen auf das Gesamtgewicht der kosmetischen Zubereitung in der zweiten Kammer erfindungsgemäß bevorzugt.

Enthält die Zubereitung Zitronensäure so ist eine Konzentration von 0,01 bis 2 Gewichts-%, bezogen auf das Gesamtgewicht der kosmetischen Zubereitung in der zweiten Kammer erfindungsgemäß bevorzugt.

Die kosmetische Zubereitung in der zweiten Kammer kann erfindungsgemäß vorteilhaft ein oder mehrere Parfümstoffe enthalten. Diese Parfümstoffe können beispielsweise gewählt werden aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C, Butylphenylmethyl-propionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Ethylenbrassylat, Evernia Furfuracea Extract, Evernia Prunastri Extract, Farnesol, Guajakholzöl, Geraniol, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Thymol, Tonkabohnenöl, Triethylcitrat und/oder Vanillin.

Die kosmetische Zubereitung in der zweiten liegt in Form einer Öl-in-Wasser Emulsion vor und umfasst Glycerylstearatcitrat, Cetearylalkohol, Natriumcetearylsulfat, Glycerylstearat, Cetearylsulfosuccinat, Natriumstearoylglutamat, Polyglyceryl-3-methylglucosedistearat, Stearinsäure, Kaliumcetylphosphat, Polyglyceryl-10 Stearat (INCI Polyglyceryl-10 Stearate) und/oder Polyclyceryl-2 Caprat enthält.

Erfindungsgemäß von Nachteil sind hingegen Zubereitungsformen auf der Basis von W/O-Emulsionen.

Erfindungsgemäß vorteilhafte Ausführungsformen der vorliegenden Erfindung sind auch dadurch gekennzeichnet, dass die kosmetische Zubereitung in der zweiten Kammer Stärke enthält.

Es ist erfindungsgemäß vorteilhaft wenn die kosmetische Zubereitung in der zweiten Kammer in der Ölphase Triglyceride und/oder Octyldodecanol enthält.

Darüber hinaus kann die Ölphase Öle aus der Gruppe der Lecithine, Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr enthalten.

Hingegen ist es nachteilig, wenn die kosmetische Zubereitung in der zweiten Kammer Silikonöle, Mineralöl und Polyethylenglycole enthält.

Erfindungsgemäß vorteilhafte Ausführungsformen der vorliegenden Erfindung sind also dadurch gekennzeichnet, dass die kosmetische Zubereitung in der zweiten Kammer frei ist von Silikonölen, Mineralöl und Polyethylenglycolen.

Um eine aus medizinischer Sicht sichere und aus kosmetischer Sicht wirksame Anwendung zu gewährleisten, ist ein ausgewogenes Verhältnis von Lyophilisat zur Wasser enthaltenden kosmetischen Zubereitung von Bedeutung.

Es ist daher erfindungsgemäß vorteilhaft, wenn das Gewichtsverhältnis von Lyophilisat zur Wasser enthaltenden kosmetischen Zubereitung 1:10 bis 1:100 beträgt.

Erfindungsgemäß bevorzugt enthält das kosmetische Produkt nach der Vermischung beider Kammerinhalte 1-10 Gewichts-% Lyophilisat und 99-90 Gewichts-% der Wasser enthaltenden kosmetischen Zubereitung.

Darüber hinaus ist es vorteilhaft im Sinne der vorliegenden Erfindung, wenn das Verhältnis von Lyophilisat zur Wasser enthaltenden kosmetischen Zubereitung so gewählt ist, dass 10⁴ bis 10⁷ Bakterien auf 2g Wasser enthaltender kosmetischer Zubereitung kommen.

Vorteilhafte Ausführungsformen der vorliegenden Erfindung sind dadurch gekennzeichnet, dass das Packmittel eine Entnahmevorrichtung und Mischungsvorrichtung enthält, mit welcher der Inhalt beider Kammern im geschlossenen System vermischt und die Mischung aus einer Öffnung entnommen werden kann.

Auch ist es erfindungsgemäß von Vorteil, wenn das Volumen der zweiten Kammer 1,5 bis 2,5 cm³ beträgt.

Als Packmittel können beispielsweise eingesetzt werden Lyo-Ject^{®} Doppelkammerspritzen, V-LK^{®} Doppelkammer-Karpullen oder Doppelkammersysteme, wie sie in der WO2018077598 A1 offenbart sind.

Erfindungsgemäß ist auch ein kosmetisches Produkt zur Verwendung in einem Verfahren zur Behandlung von Akne, dadurch gekennzeichnet, dass aus einem erfindungsgemäßen, oben beschriebenen kosmetischen Produkt der Inhalt der ersten und zweiten Kammer vermischt entnommen und in einer Menge von 0,003 bis 0,005 g/cm² auf die von Akne betroffene Haut aufgetragen werden.

Die Auftragung auf die Haut erfolgt dabei üblicherweise 0-12 Sekunden, nachdem die Inhalte beider Kammern miteinander vermischt wurden.

In diesem Verfahren ist es erfindungsgemäß vorteilhaft, wenn die von Akne betroffene Haut vor dem Auftragen des kosmetischen Produktes mit einer Tensid-haltigen Reinigungszubereitung gereinigt wird.

Auch ist es erfindungsgemäß vorteilhaft, wenn die von Akne betroffene Haut vor dem Auftragen des kosmetischen Produktes mit einer Benzoylperoxid-haltigen Zubereitung desinfiziert wird.

### Beispiel

Das nachfolgende nicht erfindungsgemäße Beispiel soll die beschriebenen Gegenstände illustrieren. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Referenzformulierungen können in Packmitteln wie Lyo-Ject^{®} Doppelkammerspritzen oder Multi-Chamber Blister (Rohrer AG) vorliegen. Im Nachfolgenden werden jeweils die Beispiele aus der ersten mit der zweiten Kammer kombiniert.

| **erste Kammer [Gewichts-%]** | | | | | |
|---|---|---|---|---|---|
| **Inhaltsstoff** | **Beispiel 1** | **Beispiel 2** | **Beispiel 3** | **Beispiel 4** | **Beispiel 5** |
| **Carageenan** | 0,50 | | | | |
| **Citrat** | | 0,17 | | | |
| **C. acnes*** | 5,00 | 1,00 | 10,00 | 8,00 | 2,00 |
| **Glycerin** | | | | | 10,00 |
| **Natriumchlorid** | | 0,90 | 0,90 | | |
| **Neutralöl** | | | 20,00 | | |
| **Octyldodecanol** | | | 10,00 | | |
| **Polyacrylsäure** | | 0,50 | | | |
| **Stärke** | | | | | 2,00 |
| **Zitronensäure** | | 0,09 | | | |

| **zweite Kammer [Gewichts-%] ohne Konservierung** | | | | | |
|---|---|---|---|---|---|
| **Carageenan** | | | | 0,20 | |
| **Citrat** | 0,17 | | | | |
| **Glycerin** | | 5,00 | | | |
| **Hyaluronsäure** | | | 1,00 | | |
| **Polyacrylsäure** | | | | | 0,20 |
| **Wasser** | 94,24 | 92,34 | 58,10 | 91,80 | 85,80 |
| **Zitronensäure** | 0,09 | | | | |

| | | | | | |
|---|---|---|---|---|---|
| * Es werden die in Anspruch 1 genannten C. *acnes* Stämme einzeln oder in Kombination in der angegebenen Menge eingesetzt. | | | | | |

## Patentansprüche

1. Kosmetisches Produkt aus einem Packmittel mit zwei räumlich getrennten Kammern, wobei die erste Kammer ein Lyophyllisat aus Bakterien der Stämme P. *acnes* 6609 (H1), C1, C3, D1, A5, H1, H2, H3, K1, K2, K4, K6, K8, K9, L1 und/oder F4 enthält und die zweite Kammer eine Wasser enthaltende kosmetische Zubereitung in Form einer Öl-in-Wasser Emulsion enthält, **dadurch gekennzeichnet, dass** die kosmetische Zubereitung in der zweiten Kammer Glycerylstearatcitrat, Cetearylalkohol, Natriumcetearylsulfat, Glycerylstearat, Cetearylsulfosuccinat, Natriumstearoylglutamat, Polyglyceryl-3-methylglucosedistearat, Stearinsäure, Kaliumcetylphosphat, Polyglyceryl-10 Stearat (INCI Polyglyceryl-10 Stearate) und/oder Polyglyceryl-2 Caprat enthält.

2. Kosmetisches Produkt nach Anspruch 1, **dadurch gekennzeichnet, dass** die kosmetische Zubereitung in der zweiten Kammer
(a) einen pH-Wert von 4 bis 8 aufweist,
(b) Carageenan und/oder Polyacrylsäure enthält,
(c) frei ist von Xanthangummi,
(d) frei ist von Parabenen, Methylisothiazolinon, Chlormethylisothiazolinon, 3-lod-2-propinylbutylcarbamat, DMDM-Hydantoin, 2-Hydroxy-4-methoxybenzophenon, 4-Methoxyzimtsäure(2-ethylhexyl)ester, 4-Methoxyzimtsäureisoamylester, 3-(4-Methylbenzyliden)campher, und/oder
(e) Glycerin, Panthenol, Ubichinon Q10 und/oder Hyaluronsäure enthält.

3. Kosmetisches Produkt nach einem der vorhergehenden Ansprüche, **dadurch gekennzeichnet, dass** die kosmetische Zubereitung in der zweiten Kammer Salicylsäure, Milchsäure und/oder Zitronensäure enthält.

4. Kosmetisches Produkt nach einem der vorhergehenden Ansprüche, **dadurch gekennzeichnet, dass** die kosmetische Zubereitung in der zweiten Kammer Triglyceride und/oder Octyldodecanol enthält.

5. Kosmetisches Produkt nach einem der vorhergehenden Ansprüche, **dadurch gekennzeichnet, dass** die kosmetische Zubereitung in der zweiten Kammer
(a) ein oder mehrere Parfümstoffe enthält,
(b) frei ist von Silikonölen, Mineralöl und Polyethylenglycolen, und/oder
(c) eine Viskosität von mindestens 500 mPas aufweist.

6. Kosmetisches Produkt nach einem der vorhergehenden Ansprüche, **dadurch gekennzeichnet, dass** das Verhältnis von Lyophilisat zur Wasser enthaltenden kosmetischen Zubereitung so gewählt ist, dass 10⁴ bis 10⁷ Bakterien auf 2g Wasser enthaltender kosmetischer Zubereitung kommen.

7. Kosmetisches Produkt nach einem der vorhergehenden Ansprüche, **dadurch gekennzeichnet, dass** in der ersten Kammer ein Lyophyllisat aus Bakterien der C. *acnes* Stämme C3 und/oder K8 eingesetzt werden.

8. Kosmetisches Produkt nach Anspruch 7, wobei die C. *acnes* Stämme C3 und K8 zu gleichen Anteilen eingesetzt werden.

9. Kosmetisches Produkt nach Anspruch 8 oder 9, **dadurch gekennzeichnet, dass** die Bakterienstämme in der ersten Kammer mit C. *acnes* Stämmen A5 und/oder F4 kombiniert werden.

10. Kosmetisches Produkt nach einem der vorhergehenden Ansprüche, **dadurch gekennzeichnet, dass**
(a) das Packmittel eine Entnahmevorrichtung und Mischungsvorrichtung enthält, mit welcher der Inhalt beider Kammern im geschlossenen System vermischt und die Mischung aus einer Öffnung entnommen werden kann, und/oder
(b) das Volumen der zweiten Kammer 1,5 bis 2,5 cm³ beträgt.

11. Kosmetisches Produkt nach einem der Ansprüche 1-10 zur Verwendung in einem Verfahren zur Behandlung von Akne, **dadurch gekennzeichnet, dass** aus dem kosmetischen Produkt der Inhalt der ersten und zweiten Kammer vermischt, entnommen und in einer Menge von 0,003 bis 0,005 g/cm² auf die von Akne betroffene Haut aufgetragen werden.

12. Kosmetisches Produkt zur Verwendung in einem Verfahren nach Anspruch 11, **dadurch gekennzeichnet, dass** die von Akne betroffene Haut vor dem Auftragen des kosmetischen Produktes mit einer Tensid-haltigen Reinigungszubereitung gereinigt wird.

13. Kosmetisches Produkt zur Verwendung in einem Verfahren nach einem der Ansprüche 11 oder 12, **dadurch gekennzeichnet, dass** die von Akne betroffene Haut vor dem Auftragen des kosmetischen Produktes mit einer Benzoylperoxid-haltigen Zubereitung desinfiziert wird.

## Claims

1. Cosmetic product comprising a packaging means with two spatially separated chambers, wherein the first chamber contains a lyophilisate of bacteria of the strains *P*. *acnes* 6609 (H1), C1, C3, D1, A5, H1, H2, H3, K1, K2, K4, K6, K8, K9, L1 and/or F4, and the second chamber contains a water-containing cosmetic preparation in the form of an oil-in-water emulsion, **characterized in that** the cosmetic preparation in the second chamber contains glyceryl stearate citrate, cetearyl alcohol, sodium cetearyl sulphate, glyceryl stearate, cetearyl sulfosuccinate, sodium stearoyl glutamate, polyglyceryl-3-methylglucose distearate, stearic acid, potassium cetyl phosphate, polyglyceryl-10 stearate (INCI polyglyceryl-10 stearate) and/or polyglyceryl-2 caprate.

2. Cosmetic product according to claim 1, **characterized in that** the cosmetic preparation in the second chamber
(a) has a pH value of 4 to 8,
(b) contains carrageenan and/or polyacrylic acid,
(c) is free from xanthan gum,
(d) is free from parabens, methylisothiazolinone, methylchloroisothiazolinone, 3-iodo-2-propynyl butylcarbamate, DMDM hydantoin, 2-hydroxy-4-methoxybenzophenone, 4-methoxycinnamic acid (2-ethylhexyl) ester, 4-methoxycinnamic acid isoamyl ester, 3-(4-methylbenzylidene)camphor, and/or
(e) contains glycerine, panthenol, ubiquinone Q10 and/or hyaluronic acid.

3. Cosmetic product according to one of the preceding claims, **characterized in that** the cosmetic preparation in the second chamber contains salicylic acid, lactic acid and/or citric acid.

4. Cosmetic product according to one of the preceding claims, **characterized in that** the cosmetic preparation in the second chamber contains triglycerides and/or octyldodecanol.

5. Cosmetic product according to one of the preceding claims, **characterized in that** the cosmetic preparation in the second chamber
(a) contains one or more perfumes,
(b) is free from silicone oils, mineral oil and polyethylene glycols, and/or
(c) has a viscosity of at least 500 mPas.

6. Cosmetic product according to one of the preceding claims, **characterized in that** the ratio of lyophilisate to water-containing cosmetic preparation is selected such that there are 10⁴ to 10⁷ bacteria per 2 g of water-containing cosmetic preparation.

7. Cosmetic product according to one of the preceding claims, **characterized in that** a lyophilisate of bacteria of C. *acnes* strains C3 and/or K8 is used in the first chamber.

8. Cosmetic product according to claim 7, wherein the C. *acnes* strains C3 and K8 are used in equal proportions.

9. Cosmetic product according to claim 8 or 9, **characterized in that** the bacterial strains in the first chamber are combined with C. *acnes* strains A5 and/or F4.

10. Cosmetic product according to one of the preceding claims, **characterized in that**
(a) the packaging means contains a dispensing device and a mixing device with which the contents of both chambers can be mixed in a closed system and the mixture can be dispensed from an opening, and/or
(b) the volume of the second chamber is 1.5 to 2.5 cm³.

11. Cosmetic product according to any of claims 1-10 for use in a method for treating acne, **characterized in that** the contents of the first and second chambers of the cosmetic product are mixed, dispensed and applied in an amount of 0.003 to 0.005 g/cm² to the skin affected by acne.

12. Cosmetic product for use in a method according to claim 11, **characterized in that** the skin affected by acne is cleansed with a surfactant-containing cleansing preparation before the cosmetic product is applied.

13. Cosmetic product for use in a method according to one of claims 11 or 12, **characterized in that** the skin affected by acne is disinfected with a benzoyl peroxide-containing preparation before applying the cosmetic product.

## Revendications

1. Produit cosmétique composé d'un moyen de conditionnement avec deux compartiments spatialement séparés, dans lequel le premier compartiment contient un lyophilisat de bactéries des souches *P*. *acnes* 6609 (H1), C1, C3, D1, A5, H1, H2, H3, K1, K2, K4, K6, K8, K9, L1 et/ou F4, et le deuxième compartiment contient une préparation cosmétique contenant de l'eau sous la forme d'une émulsion huile dans l'eau, **caractérisé en ce que** la préparation cosmétique dans le deuxième compartiment contient du citrate de stéarate de glycéryle, de l'alcool cétéarylique, du sulfate de cétéaryle de sodium, du stéarate de glycéryle, du sulfosuccinate de cétéaryle, du stéaroyl glutamate de sodium, du distéarate polyglycéryl-3-méthylglucose, de l'acide stéarique, du phosphate de cétyle de potassium, du polyglycéryl-10-stéarate (INCI Polyglyceryl-10 Stearate) et/ou du polyglycéryle-2-caprate.

2. Produit cosmétique selon la revendication 1, **caractérisé en ce que** la préparation cosmétique dans le deuxième compartiment
(a) présente un pH de 4 à 8,
(b) contient du carraghénane et/ou de l'acide polyacrylique,
(c) est exempte de caoutchouc xanthane,
(d) est exempte de parabènes, de méthylisothiazolinone, de chlorméthylisothiazolinone, de 3-iode-2-propinylbutylcarbamate, de DMDM-hydantoïne, de 2-hydroxy-4-méthoxybenzophénone, d'ester 2-éthylhexyle d'acide 4-méthoxycinnamique, d'ester d'isoamyle d'acide 4-méthoxycinnamique, de 3-(4-méthylbenzylidène)camphre, et/ou
(e) contient de la glycérine, du panthénol, de l'ubiquinone Q10 et/ou de l'acide hyaluronique.

3. Produit cosmétique selon l'une quelconque des revendications précédentes, **caractérisé en ce que** la préparation cosmétique contient dans le deuxième compartiment de l'acide salicylique, de l'acide lactique et/ou de l'acide citrique.

4. Produit cosmétique selon l'une des revendications précédentes, **caractérisé en ce que** la préparation cosmétique contient dans le deuxième compartiment des triglycérides et/ou de l'octyldodécanol.

5. Produit cosmétique selon l'une des revendications précédentes, **caractérisé en ce que** la préparation cosmétique dans le deuxième compartiment
(a) contient un ou plusieurs parfums,
(b) est exempte d'huiles de silicone, d'huile minérale et de polyéthylène glycols, et/ou
(c) présente une viscosité d'au moins 500 mPas.

6. Produit cosmétique selon l'une quelconque des revendications précédentes, **caractérisé en ce que** le rapport entre le lyophilisat et la préparation cosmétique contenant de l'eau est choisi de manière à obtenir entre 10⁴ et 10⁷ bactéries pour 2 g de préparation cosmétique contenant de l'eau.

7. Produit cosmétique selon l'une quelconque des revendications précédentes, **caractérisé en ce qu'**un lyophilisat de bactéries des souches *C*. *acnes* C3 et/ou K8 est employé dans le premier compartiment.

8. Produit cosmétique selon la revendication 7, dans lequel les souches *C*. *acnes* C3 et K8 sont employées en proportions égales.

9. Produit cosmétique selon la revendication 8 ou 9, **caractérisé en ce que** les souches bactériennes dans le premier compartiment sont combinées à des souches de C. *acnes* A5 et/ou F4.

10. Produit cosmétique selon l'une quelconque des revendications précédentes, **caractérisé en ce que**
(a) le moyen de conditionnement contient un dispositif de prélèvement et un dispositif de mélange avec lesquels le contenu des deux compartiments peut être mélangé dans le système fermé et le mélange peut être prélevé à partir d'une ouverture, et/ou
(b) le volume du deuxième compartiment va de 1,5 à 2,5 cm 3.

11. Produit cosmétique selon l'une quelconque des revendications 1 à 10, destiné à être utilisé dans un procédé de traitement de l'acné, **caractérisé en ce que** du produit cosmétique, le contenu des premier et deuxième compartiments est mélangé, prélevé et appliqué sur la peau affectée par l'acné en une quantité de 0,003 à 0,005 g/cm².

12. Produit cosmétique destiné à être utilisé dans un procédé selon la revendication 11,
**caractérisé en ce que** la peau affectée par l'acné est nettoyée avant l'application du produit cosmétique avec une préparation nettoyante contenant des tensioactifs.

13. Produit cosmétique destiné à être utilisé dans un procédé selon l'une quelconque des revendications 11 ou 12, **caractérisé en ce que** la peau affectée par l'acné est désinfectée avec une préparation contenant du peroxyde de benzoyle avant l'application du produit cosmétique.
